# EUROPEAN PATENT APPLICATION

(11) **EP 4 623 857 A1**
(43) Date of publication of application: **01.10.2025**
(21) Application number: 24883587.8
(22) Date of filing: 27.12.2024
(51) Int. Cl.: A61C 7/00

(54) **METHOD FOR GENERATING EDGE CUTTING LINES OF ORTHODONTIC ALIGNER, ORTHODONTIC ALIGNER, AND READABLE MEDIUM**

(30) Priority: 29.12.2023 CN 202311871847
(71) Applicant: Wuxi EA Bio-Tech Co., Ltd., Wuxi, Jiangsu 214000 (CN)
(72) Inventor: WANG, Meng, Wuxi, Jiangsu 214000 (CN); LI, Junsheng, Wuxi, Jiangsu 214000 (CN); ZHENG, Yikan, Wuxi, Jiangsu 214000 (CN)
(74) Representative: Winter, Brandl - Partnerschaft mbB
(86) International application number: PCT/CN2024/143253
(87) International publication number: WO 2025/140583

(57) **Abstract**

The present application discloses a method for generating an edge cutting line for an orthodontic aligner, an orthodontic aligner, and a computer-readable medium in the field of orthodontic treatment. The generating method includes: identifying a tooth contour of a dental model corresponding to at least one first tooth, and obtaining a first region corresponding to the tooth contour; determining a second region corresponding to an attachment, wherein the attachment is located at least partially on the orthodontic aligner corresponding to at least a portion of the at least one first tooth or an interdental space, wherein the orthodontic aligner is related to the dental model; when the first region cannot completely cover the second region, generating an edge cutting line for the orthodontic aligner based on the second region, wherein the edge cutting line is configured to cut the orthodontic aligner. Through such arrangement, sufficient space is provided on the finally cut orthodontic aligner for adding the attachment, which improves the success rate of adding the attachment on the orthodontic aligner, thereby enabling the attachment to better cooperate with the orthodontic aligner for orthodontic correction of teeth.

## Description

### TECHNICAL FIELD

The present application relates to the field of orthodontic treatment, and particularly relates to a method for generating an edge cutting line for an orthodontic aligner, an orthodontic aligner and a computer-readable medium.

### BACKGROUND

Currently, orthodontic aligners function by wrapping around crowns and providing retention. However, during invisible orthodontic treatment, to achieve the expected treatment effects, various attachments often need to be designed on the orthodontic aligner. When adding attachments to the orthodontic aligner, a certain area for addition is required, which is generally on a buccal surface, a lingual surface, or an occlusal surface. Most attachments are added to the buccal and lingual surfaces, where the available area for addition is generally limited to the region between the orthodontic aligner edge and the occlusal incisal edge, which may result in failure to achieve the expected treatment effects.

### SUMMARY

The technical problem to be solved by the present application is how to improve the success rate of adding attachments to an orthodontic aligner, and to provide a method for generating an edge cutting line for an orthodontic aligner, an orthodontic aligner and a computer-readable medium. The present application solves the above technical problem through the following technical solution:
The present application provides a method for generating an edge cutting line for an orthodontic aligner, including:
identifying a tooth contour of a dental model corresponding to at least one first tooth, and obtaining a first region corresponding to the tooth contour; determining a second region corresponding to an attachment, wherein the attachment is located at least partially on the orthodontic aligner corresponding to at least a portion of the at least one first tooth or an interdental space, wherein the orthodontic aligner is related to the dental model; when the first region cannot completely cover the second region, generating an edge cutting line for the orthodontic aligner based on the second region, wherein the edge cutting line is configured to cut the orthodontic aligner.

In this solution, by comparing the obtained first region with the second region, a generation reference for the edge cutting line of the orthodontic can be determined based on a comparison result, making an edge cutting of the orthodontic aligner more flexible and adaptable to various types of attachments. When the first region cannot completely cover the second region, the edge cutting line of the orthodontic aligner is directly generated based on the second region, ensuring that the finally cut orthodontic aligner has sufficient space for adding attachments, which improves the success rate of adding attachments to the orthodontic aligner, thereby enabling the attachments to better cooperate with the orthodontic aligner for orthodontic correction of teeth.

In one possible implementation, the edge cutting line satisfies at least one of the following:
at least a portion of the edge cutting line is located below a first gingival line;
at least a portion of the edge cutting line intersects with the first gingival line;
at least a portion of the second region is located below the first gingival line;
at least a portion of the second region intersects with the first gingival line;
wherein the first gingival line includes a gingival line of the at least one first tooth, and/or at least a portion of a gingival line of at least one tooth adjacent to the at least one first tooth.

In one possible implementation, further includes:
when there is occlusal interference between the attachment and an opposing tooth of the first tooth, adjusting a position of the attachment on the orthodontic aligner to a first position.

In one possible implementation, before adjusting the position of the attachment on the orthodontic aligner to the first position, the attachment is at a second position on the orthodontic aligner, wherein the second position is a position where the attachment is located when there is occlusal interference between the attachment and the opposing tooth of the first tooth.

In one possible implementation, a priority of the first position is lower than or equal to a priority of the second position.

In one possible implementation, the priority of the position where the attachment is located is related to function information of the attachment.

In one possible implementation, further includes:
when there is occlusal interference between the attachment and the opposing tooth of the first tooth, adjusting the position of the attachment on the orthodontic aligner, and the direction of adjustment is toward a gingival margin.

In one possible implementation, before generating the edge cutting line, there is no occlusal interference between the attachment and the opposing tooth of the first tooth.

In one possible implementation, the edge cutting line is determined at a preset distance away from position information of the second region.

In one possible implementation, the preset distance ranges from 0.2mm to 2mm.

In one possible implementation, the edge cutting line extends along a first direction of the first tooth, and at least covers at least one tooth adjacent to the at least one first tooth or an interdental space.

In one possible implementation, further includes:
displaying at least a portion of the dental model;
displaying at least one of the edge cutting line, the attachment, and the orthodontic aligner.

In one possible implementation, further includes:
obtaining a first instruction from a user, wherein the first instruction is configured to indicate desired position information of the attachment or desired position information of an orthodontic aligner edge;
wherein generating the edge cutting line based on the second region includes: generating a first cutting line based on the first instruction and the second region.

In one possible implementation, further includes:
in response to a second instruction from the user, adjusting the generated first cutting line to a second cutting line, wherein the second instruction is configured to indicate adjustment information of the first cutting line, and the second cutting line is configured to generate a target orthodontic aligner.

In one possible implementation, the second instruction is configured to indicate at least one of:
updating position information of the attachment;
an adjustment target position of the first cutting line.

The present application further provides a method for generating an edge cutting line for an orthodontic aligner, including:
obtaining a dental model corresponding to at least one first tooth;
in response to a first instruction from a user and the dental model, determining a second region corresponding to an attachment, wherein the attachment is located at least partially on an orthodontic aligner corresponding to at least a portion of the at least one first tooth or an interdental space; wherein the first instruction is configured to indicate position information of the attachment;
generating an edge cutting line based on the second region.

In one possible implementation, the edge cutting line satisfies at least one of the following:
at least a portion of the edge cutting line is located below a first gingival line;
at least a portion of the edge cutting line intersects with the first gingival line;
at least a portion of the second region is located below the first gingival line;
at least a portion of the second region intersects with the first gingival line;
wherein the first gingival line includes a gingival line of the at least one first tooth, and/or at least a portion of a gingival line of at least one tooth adjacent to the at least one first tooth.

In one possible implementation, further includes:
when there is occlusal interference between the attachment and an opposing tooth of the first tooth, adjusting a position of the attachment on the orthodontic aligner to a first position.

In one possible implementation, before adjusting the position of the attachment on the orthodontic aligner to the first position, the attachment is at a second position on the orthodontic aligner, wherein the second position is a position where the attachment is located when there is occlusal interference between the attachment and the opposing tooth of the first tooth.

In one possible implementation, a priority of the first position is lower than or equal to a priority of the second position.

In one possible implementation, the priority of the position where the attachment is located is related to function information of the attachment.

In one possible implementation, further includes:
when there is occlusal interference between the attachment and the opposing tooth of the first tooth, adjusting the position of the attachment on the orthodontic aligner, and the direction of adjustment is toward a gingival margin.

In one possible implementation, before generating the edge cutting line, there is no occlusal interference between the attachment and the opposing tooth of the first tooth.

In one possible implementation, the edge cutting line is determined at a preset distance away from position information of the second region.

In one possible implementation, the preset distance ranges from 0.2mm to 2mm.

In one possible implementation, the edge cutting line extends along a first direction of the first tooth, and at least covers at least one tooth adjacent to the at least one first tooth or an interdental space.

In one possible implementation, further includes:
displaying at least a portion of the dental model;
displaying at least one of the edge cutting line, the attachment, and the orthodontic aligner.

In one possible implementation, in response to a second instruction from the user, adjusting the generated first cutting line to a second cutting line, wherein the second instruction is configured to indicate adjustment information of the first cutting line, and the second cutting line is configured to generate a target orthodontic aligner.

In one possible implementation, the second instruction is configured to indicate at least one of:
updating position information of the attachment;
an adjustment target position of the first cutting line.

The present application further provides an orthodontic aligner configured to be fitted on teeth, the orthodontic aligner includes an aligner body and an attachment mounted on the aligner body;
wherein an edge of the orthodontic aligner is determined based on any of the aforementioned methods for generating an edge cutting line for an orthodontic aligner.

The present application further provides a computer-readable medium, the computer-readable medium stores instructions executable by a processor to cause a computing device to perform a method, wherein the method includes any of the aforementioned methods for generating an edge cutting line for an orthodontic aligner.

The present application provides an orthodontic aligner, including: an aligner body and an attachment mounted on the aligner body, wherein the aligner body includes a second region for adding the attachment, and when the orthodontic aligner is fitted on teeth, a portion of the second region is located below a first gingival line of teeth and another portion is located above the first gingival line of teeth.

In one possible implementation, when the orthodontic aligner is fitted on teeth, a portion of a bottom surface of the attachment connecting with the second region corresponds to a region below the first gingival line, and another portion corresponds to a region above the first gingival line.

In one possible implementation, when the orthodontic aligner is fitted on teeth, the second region corresponds to a crown of at least one first tooth or an interdental space, when there is one first tooth, the second region corresponds to the crown and gingiva of the first tooth, when there are multiple first teeth, the second region corresponds to crowns, gingiva, and interdental spaces of the multiple first teeth.

In one possible implementation, when the orthodontic aligner is fitted on teeth, the attachment is located at least partially on the orthodontic aligner corresponding to at least a portion of the at least one first tooth or an interdental space, when there is one first tooth, the attachment corresponds to the crown and gingiva of the first tooth, when there are multiple first teeth, the attachment corresponds to crowns, gingiva, and interdental spaces of the multiple first teeth.

In one possible implementation, the orthodontic aligner includes an edge cutting line, wherein the edge cutting line is located below the first gingival line only at the portion of the first tooth, and the edge cutting line substantially coincides with the first gingival line at portions of other teeth.

In one possible implementation, the orthodontic aligner includes an edge cutting line, wherein the edge cutting line is located below the first gingival line at the portion of the first tooth and portions of other teeth adjacent to the first tooth.

In one possible implementation, the orthodontic aligner includes an edge cutting line, and a preset distance between the edge cutting line and an outer contour line of the corresponding second region ranges from 0.2mm to 2mm.

In one possible implementation, when the orthodontic aligner is fitted on teeth and in occlusion with opposing teeth, the attachment is spaced apart from the opposing teeth.

In one possible implementation, the teeth include crowns located above the first gingival line and gingiva located below the first gingival line, wherein the first end portion corresponds to the crown and there is the first distance between the first end portion and the crown, and the second end portion corresponds to the gingiva and there is the second distance between the second end portion and the gingiva.

In one possible implementation, the second end portion is tightly fitted to the gingiva.

In one possible implementation, the aligner body further includes a third region and a connecting region connecting the third region and the second region, wherein the third region is tightly fitted to the crown, and the connecting region forms a bend between the second region and the third region.

In one possible implementation, the second region is a plane.

In one possible implementation, an angle between the plane of the second region and the occlusal plane ranges from 60° to 90°.

In one possible implementation, a bottom surface of the attachment connects with the second region, and the bottom surface covers part or all of the second region.

In one possible implementation, the attachment is a traction buckle, a traction hook, a tubular attachment, or a groove-shaped attachment.

The present application provides an orthodontic aligner, the orthodontic aligner includes an aligner body and an attachment mounted on the aligner body, wherein the aligner body includes a second region for adding the attachment, when the orthodontic aligner is fitted on teeth, the second region is close to a first gingival line of teeth, or the second region intersects with the first gingival line, in a direction perpendicular to an occlusal plane, the second region incluides oppositely disposed first end portion and second end portion, there is a first distance between the first end portion and the teeth, and there is a second distance between the second end portion and the teeth, wherein the first distance is greater than the second distance.

In one possible implementation, a portion of the second region is located below the first gingival line of teeth and another portion is located above the first gingival line of teeth; or the second region is entirely located below the first gingival line; or the second region is entirely located above the first gingival line.

In one possible implementation, the teeth include crowns located above the first gingival line and gingiva located below the first gingival line, wherein the first end portion corresponds to the crown and there is the first distance between the first end portion and the crown, and the second end portion corresponds to the gingiva and there is the second distance between the second end portion and the gingiva.

In one possible implementation, the second end portion is tightly fitted to the gingiva.

In one possible implementation, the aligner body further includes a third region and a connecting region connecting the third region and the second region, wherein the third region is tightly fitted to the crown, and the connecting region forms a bend between the second region and the third region.

In one possible implementation, the second region is a plane.

In one possible implementation, an angle between the plane of the second region and the occlusal plane ranges from 60° to 90°.

In one possible implementation, a bottom surface of the attachment connects with the second region, and the bottom surface covers part or all of the second region.

In one possible implementation, the attachment is a traction buckle, a traction hook, a tubular attachment, or a groove-shaped attachment.

In one possible implementation, the second region partially extends below the first gingival line, the orthodontic aligner includes an edge cutting line, and a preset distance between the edge cutting line and an outer contour line of the corresponding second region ranges from 0.2mm to 2mm.

In one possible implementation, the second region partially extends below the first gingival line, the orthodontic aligner includes an edge cutting line, wherein the edge cutting line is located below the first gingival line only at the portion of the second region, and other portions of the edge cutting line substantially coincide with the first gingival line.

In one possible implementation, the second region partially extends below the first gingival line, the orthodontic aligner includes an edge cutting line, wherein the edge cutting line is located below the first gingival line at the portion of the second region and portions of other teeth adjacent to the second region.

In one possible implementation, when the orthodontic aligner is fitted on teeth and in occlusion with opposing teeth, the attachment is spaced apart from the opposing teeth.

### BRIEF DESCRIPTION OF THE DRAWINGS

FIG. 1 is a schematic diagram showing the fitting relationship between an orthodontic aligner without attachment and teeth according to an embodiment of the present application.
FIG. 2 is a first schematic diagram showing the fitting relationship between an orthodontic aligner with attachment and teeth according to an embodiment of the present application.
FIG. 3 is a second schematic diagram showing the fitting relationship between an orthodontic aligner with attachment and teeth according to an embodiment of the present application.
FIG. 4 is a flow diagram of a method for generating an edge cutting line for an orthodontic aligner according to an embodiment of the present application.
FIG. 5 is a first schematic diagram showing the fitting relationship between an orthodontic aligner with attachment and teeth according to an embodiment of the present application.
FIG. 6 is a second schematic diagram showing the fitting relationship between an orthodontic aligner with attachment and teeth according to an embodiment of the present application.
FIG. 7 is a third schematic diagram showing the fitting relationship between an orthodontic aligner with attachment and teeth according to an embodiment of the present application.
FIG. 8 is a structural schematic diagram of an electronic device according to an embodiment of the present application;
FIGs. 9 to 13 are schematic diagrams showing the fitting relationship between an orthodontic aligner and teeth according to an embodiment of the present application;
FIGs. 14 to 17 are schematic diagrams showing the fitting relationship between an orthodontic aligner and teeth according to another embodiment of the present application;

### Reference Numerals:

Orthodontic aligner 10
Crown 20
Gingival line 30
Edge cutting line 40
Attachment 50
Opposing tooth 60
Second region 70
First end portion 71
Second end portion 72
Aligner body 101
Bottom surface 51
Traction member 52
Bottom portion 53
Connecting rod 54
Top portion 55

### DETAILED DESCRIPTION

The embodiments of the present application will now be described in detail. The examples of the embodiments are shown in the accompanying drawings, wherein the same or similar reference numerals throughout denote the same or similar elements or elements having the same or similar functions. The embodiments described below with reference to the drawings are exemplary and are intended to explain the present application rather than to be understood as limitations of the present application. All other embodiments obtained by persons skilled in the art without creative effort based on the embodiments of the present application fall within the scope of protection of the present application.

In the description of the present application, it should be understood that terms such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "circumferential", "radial" and other orientations or positional relationships are based on the orientations or positional relationships shown in the drawings, are only for the purpose of describing the present application and simplifying the description, and do not indicate or imply that the referred device or element must have a specific orientation, be constructed in a specific orientation and operate in a specific orientation, therefore should not be understood as limitations of the present application.

Furthermore, the terms "first", "second" are only used for descriptive purposes and should not be understood as indicating or implying relative importance or implicitly indicating the number of technical features referred to. Thus, features defined as "first", "second" may explicitly or implicitly include one or more of these features. In the description of the present application, "multiple" means two or more unless explicitly specified otherwise.

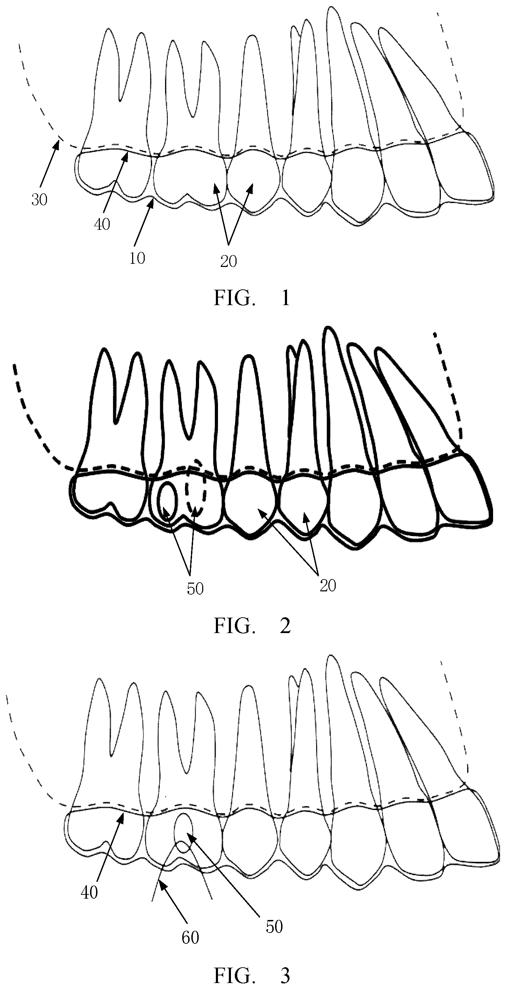

As shown in FIG. 1, in one possible solution, an edge of an orthodontic aligner 10 can be cut along a gingival line 30, that is, an edge cutting line 40 is generally generated at a position of the gingival line 30, and the orthodontic aligner 10 is fitted on crowns 20 of teeth.

As shown in FIG. 2, when the height of the crowns 20 of the teeth is relatively low, an area available for adding an attachment 50 will be relatively small in the vertical direction of the teeth, which may not meet space dimension requirements needed for adding the attachment 50, resulting in failure to add the attachment 50 or a low success rate of addition.

For example, as shown in FIG. 3, when there is occlusal interference between the added attachment 50 and opposing teeth, it needs to be moved further toward the gingival line 30 direction, but due to a limitation of the edge cutting line 40, there is no space needed for further movement, thus resulting in unsuccessful addition of the attachment 50.

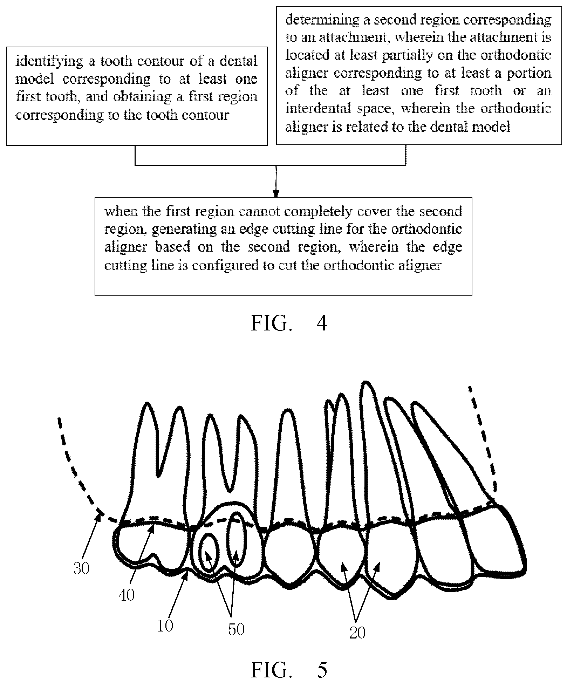

Based on problems existing in the above solution, this embodiment provides a method for generating the edge cutting line 40 for the orthodontic aligner. As shown in FIGs. 5, 6, and 7, the orthodontic aligner 10 has the attachment 50 added thereto. Depending on the type of attachment 50, the methods of adding the attachment 50 to the orthodontic aligner 10 vary. For example, the attachment 50 can be added to the orthodontic aligner 10 through fixed connection methods such as adhesion, or can be integrally formed on the orthodontic aligner 10 (such as tongue spurs), or can be added to the orthodontic aligner 10 through other methods. It should be noted that in the field of orthodontic treatment, there are many types of attachments 50 for auxiliary orthodontic devices to move teeth, including those added to the orthodontic aligner 10 and those added directly to the teeth. The attachment 50 in this embodiment only refers to attachments 50 that are added to the orthodontic aligner 10.

Since the orthodontic aligner 10 is fitted on the user's teeth during use, data can first be collected from the user's teeth to generate a dental model corresponding to the user's teeth. Then, through digital simulation, an orthodontic aligner 10 in its initial state can be simulated to fit on the dental model, where the orthodontic aligner 10 in the initial state can be one that is cut along the gingival line 30. The dental model can be generated based on a three-dimensional model obtained by scanning the user's oral cavity, or based on two-dimensional images formed by photographing the user's oral cavity, or based on various types of image data obtained after scanning dental impression data. Meanwhile, regarding the data source of the dental model, it can be collected before generating the treatment plan, during the treatment process when updating the treatment plan, or during remote monitoring.

On this basis, as shown in FIG. 4, a tooth contour of the dental model corresponding to at least one first tooth is identified, and a first region corresponding to the tooth contour is obtained. It should be noted that teeth in the oral cavity are generally divided into root and crown 20, with the root surrounded by gingiva. The tooth contour corresponds to the external contour formed by the combination of the crown 20 of the first tooth and interdental spaces, which is a three-dimensional shape. When the attachment 50 is added to the orthodontic aligner 10 and works with the orthodontic aligner 10 for orthodontic correction, the attachment 50 is generally located on a labial side or a lingual side of the teeth. Therefore, the first region corresponding to the tooth contour is the area corresponding to the tooth contour on the labial or lingual side of the teeth.

In this embodiment, the first tooth is a tooth on the dental model. The number of first teeth can be one or multiple. If there is one first tooth, then the first tooth is the specific tooth that the attachment 50 on the orthodontic aligner 10 acts upon. In this case, the identified tooth contour is an external contour of the crown 20 corresponding to the specific tooth, and the first region obtained is an area corresponding to the external contour on the labial or lingual side of the tooth. If there are multiple first teeth, the attachment 50 may correspond to one of the first teeth on the orthodontic aligner 10, or may correspond to multiple first teeth and interdental spaces on the orthodontic aligner 10. In this case, the identified tooth contour is the external contour formed by the combination of the crowns 20 of multiple first teeth and interdental spaces, and the first region obtained is the area corresponding to the external contour on the labial or lingual side of the teeth.

While obtaining the first region, on the orthodontic aligner 10 in the initial state, a second region corresponding to where the attachment 50 is to be added is determined. Although the orthodontic aligner 10 in the initial state is simulated to fit on the dental model, and the attachment 50 is added to the orthodontic aligner 10, when the orthodontic aligner 10 is fitted on the dental model, at least a portion of the attachment 50 also corresponds to at least a portion of the first tooth or interdental spaces. At this time, the determination of the second region can be made according to an area occupied by the attachment 50 relative to the first tooth. It should be noted that when there is one first tooth, the attachment 50 may directly correspond to the crown 20 of the first tooth, or may correspond to both the crown 20 and gingiva of the first tooth. When there are multiple first teeth, the attachment 50 may correspond to the crowns 20 and interdental spaces of multiple first teeth, or may correspond to the crowns 20, gingiva, and interdental spaces of multiple first teeth.

Specifically, the determination of the second region can be made based on the type of attachment 50. For example, when the type of attachment 50 is determined, its corresponding position on the first tooth can be determined, and then the second region can be directly determined based on a coverage area of the attachment 50 on the first tooth. If the position of the first tooth that the attachment 50 needs to correspond to cannot be determined solely based on the type of attachment 50, then the second region can be determined based on both the type of attachment 50 and its intended position of action. If the environment where the attachment 50 is added may affect the determination of the second region, then in addition to the type of attachment 50 and its intended position of action, the environment where the attachment 50 is located should also be considered. The environment specifically includes: individual crown 20, interdental space, gingiva, etc., or a combination of the above.

When both the first region and the second region are determined, they are compared. If the first region can completely cover the second region, for example, as shown in a left side of FIG. 5, where there is one first tooth and the attachment 50 does not correspond to the gingiva of the first tooth, this indicates that when the orthodontic aligner 10 is fitted on the user's teeth, the attachment 50 on the orthodontic aligner 10 is located within the first region. In this case, the edge cutting line 40 can be directly generated at the position of the gingival line 30, and the edge cutting line 40 can be used to cut the orthodontic aligner 10. When the cut orthodontic aligner 10 is fitted on the user's teeth, the attachment 50 on the orthodontic aligner 10 can directly act on the user's teeth without issues of attachment addition failure or low success rate.

In some embodiments, if the first region cannot completely cover the second region, for example, as shown in a right side of FIG. 5, where there is one first tooth and the attachment 50 partially corresponds to the gingiva of the first tooth, the edge cutting line 40 can be generated based on the second region, and the orthodontic aligner 10 can be cut according to this edge cutting line 40. When the orthodontic aligner 10 is fitted on the user's teeth, the attachment 50 can not only be successfully added to the orthodontic aligner 10 but also achieve a higher success rate of addition.

Specifically, when the first region cannot completely cover the second region, there can be multiple scenarios for the form of the second region and the edge cutting line 40. The following examples illustrate scenarios 1 to 4.

Scenario 1: The portion of the edge cutting line 40 corresponding to the first tooth is located below the first gingival line 30, where the first gingival line 30 includes the gingival line 30 of the first tooth.

It should be understood that the gingival line 30 serves as a boundary line for teeth, where above the gingival line 30 is the direction of the tooth crown 20, and below the gingival line 30 is the direction of the gingiva. Through this arrangement, after cutting the orthodontic aligner 10 using the generated edge cutting line 40, when the orthodontic aligner 10 is fitted on the user's teeth, the attachment 50 can be successfully added to the orthodontic aligner 10 and act on the user's teeth.

As shown in FIG. 5, there is one first tooth, and the first gingival line 30 includes not only the gingival line 30 of this first tooth but also the gingival lines 30 of multiple teeth adjacent to the first tooth. However, the generated edge cutting line 40 is located below the first gingival line 30 only at the position of the first tooth and coincides with the first gingival line 30 at the positions of other teeth. However, in other embodiments, the number of first teeth can be one or multiple, and the first gingival line 30 can include only the gingival line 30 of the first tooth, or the first gingival line 30 can include not only the gingival line 30 of the first tooth but also at least a portion of the gingival line 30 of at least one tooth adjacent to the first tooth. Meanwhile, the edge cutting line 40 can also be located below the first gingival line 30 at part of the first tooth's position rather than corresponding to the entire position of the first tooth; and the edge cutting line 40 can also be located below the first gingival line 30 at parts of both the first tooth and an adjacent tooth. There are no fixed restrictions, as long as when the cut orthodontic aligner 10 is fitted on the user's teeth, the attachment 50 can be successfully added to the orthodontic aligner 10.

Scenario 2: At least a portion of the edge cutting line 40 intersects with the first gingival line 30, where the first gingival line 30 includes the gingival line 30 of the first tooth.

As shown in FIG. 5, there is one first tooth, and the first gingival line 30 includes not only the gingival line 30 of the first tooth but also the gingival lines 30 of multiple teeth adjacent to the first tooth. However, the generated edge cutting line 40 has portions that intersect with the first gingival line 30, that is, the edge cutting line 40 is located below the first gingival line 30 only at the position of the first tooth, so that when the orthodontic aligner 10 is fitted on the user's teeth, the attachment 50 can be successfully added to the orthodontic aligner 10 and act on the user's teeth.

Scenario 3: A portion of the second region is located below the first gingival line 30.

As shown in FIG. 5, the second region corresponding to the attachment 50 on the right side of the first tooth is partially located below the first gingival line 30. At this time, the corresponding first region cannot cover the second region, so the edge cutting line 40 can be generated based on the second region, and the orthodontic aligner 10 can be cut according to the edge cutting line 40, ensuring that when the orthodontic aligner 10 is fitted on the user's teeth, the attachment 50 can be successfully added to the orthodontic aligner 10 and act on the user's teeth. It should be noted that in other embodiments, the second region may also be entirely located below the first gingival line 30.

Scenario 4: A portion of the second region intersects with the first gingival line 30.

As shown in FIG. 5, the second region corresponding to the attachment 50 on the right side of the first tooth partially intersects with the first gingival line 30. At this time, the corresponding first region also cannot cover the second region, so similarly, the edge cutting line 40 can be generated based on the second region, and the orthodontic aligner 10 can be cut according to the edge cutting line 40.

It should be noted that the above four scenarios may have partial overlaps or combinations, that is, in specific implementations, combinations of two, three, or even all four scenarios can be adopted.

In one possible implementation, when the attachment 50 is added to the orthodontic aligner 10 and works with the orthodontic aligner 10 for orthodontic treatment, there may be occlusal interference between the attachment 50 and the opposing tooth 60 of that tooth. Therefore, it is necessary to adjust the position of the attachment 50 on the orthodontic aligner 10 to avoid occlusal interference while ensuring the function of the attachment 50.

Thus, in this method for generating the edge cutting line 40, when comparing the first region and the second region, it can also be determined whether there would be occlusal interference between the attachment 50 on the orthodontic aligner 10 and the opposing tooth 60 of the first tooth. If there is occlusal interference, the position of the attachment 50 on the orthodontic aligner 10 should be adjusted accordingly.

There can be multiple implementations for the adding position. The following example uses addition at a first position.

The first position can be an optimal position chosen based on practical application, a position chosen based on the patient's symptoms, or a position chosen based on the patient's dental condition. This is not limited.

When the attachment 50 is added to the orthodontic aligner 10 at the first position, it can be determined whether there would be occlusal interference between the attachment 50 and the opposing tooth 60 of the first tooth. Meanwhile, it can also be determined whether the first region can cover the second region. When there is no occlusal interference and the first region can cover the second region, the edge cutting line 40 can be generated at the position of the gingival line 30, or when there is no occlusal interference but the first region cannot completely cover the second region, the edge cutting line 40 can be generated based on the second region. It should be noted that the first position where the attachment 50 is added to the orthodontic aligner 10 is determined based on the type of attachment 50, and the attachment 50 can maximize its function at the first position.

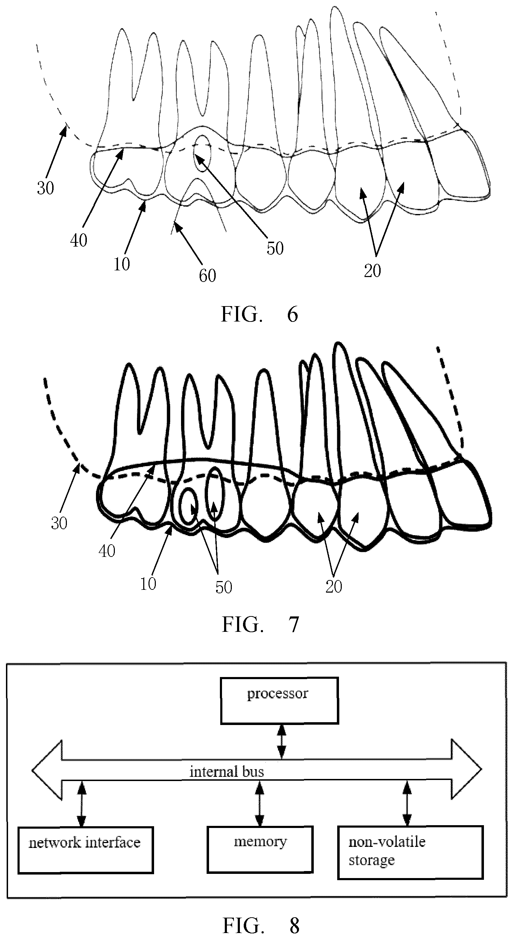

However, if there would be occlusal interference when the attachment 50 is added to the orthodontic aligner 10 at the first position, the position of the attachment 50 on the orthodontic aligner 10 needs to be adjusted to the first position. As shown in FIG. 6, the arrow direction in the figure indicates the direction of adjustment for the attachment 50, and the position of the attachment 50 shown in the figure is the adjusted first position, where there would be no occlusal interference with the opposing tooth 60 of the first tooth.

It should be noted that when the position of the attachment 50 on the orthodontic aligner 10 changes, the corresponding second region will also change. Therefore, there is no sequential order between the step of "comparison between the first region and the second region" and the step of "determining whether there would be occlusal interference between the attachment 50 added to the orthodontic aligner 10 and the opposing tooth 60 of the first tooth". It should be understood that these two steps are performed in an overlapping manner, which is not limited here. The goal is to ensure that when the orthodontic aligner 10 is finally fitted on the user's teeth, the attachment 50 can be successfully added to the orthodontic aligner 10 and work with the orthodontic aligner 10 for orthodontic correction while avoiding occlusal interference between the attachment 50 and the opposing tooth 60 of that tooth.

Furthermore, positions where attachments 50 are added to the orthodontic aligner 10 have priorities, and attachments 50 are added to the orthodontic aligner 10 according to these priority levels. The priorities are specifically determined based on characteristics such as the shape of the attachment 50 and the function the attachment 50 is intended to achieve. For example, for a specific attachment 50, top ten priority positions for addition are listed, where a first-ranked position is the first position mentioned above, and when the attachment 50 is added at the first position, it can maximize its functionality. In terms of priority, the second-ranked position is lower than the first-ranked position, meaning that when the attachment 50 is added at the second-ranked position, its achievable functionality is less than when added at the first-ranked position; similarly, this applies to a third-ranked position, a fourth-ranked position, and so on. Of course, the same priority level may correspond to two or more positions. In this case, based on the need to better achieve the attachment's 50 function, any of these two or more positions with the same priority level can be used to add the attachment 50. However, in such cases, other factors can also be considered to decide which position the attachment 50 should be added to, specifically referring to factors that exist in practical application scenarios, which will not be elaborated here.

As shown in FIG. 6, when the attachment 50 is added at the highest priority position, there may be occlusal interference with the opposing tooth 60 of the first tooth, requiring a downgrade in the installation position of the attachment 50. For example, when the attachment 50 installed at a first-level position would have occlusal interference with the opposing tooth 60 of the first tooth, the attachment 50 is adjusted to a second-level position. When the attachment 50 installed at the second-level position would still have occlusal interference with the opposing tooth 60 of the first tooth, the installation position of the attachment 50 can continue to be downgraded, for example, to a third-level position, and so on. Through this priority-based sequential downgrading, the position of the attachment 50 on the orthodontic aligner 10 is adjusted until reaching the first position, where the attachment 50 will not have occlusal interference with the opposing tooth 60 of the first tooth.

It should be noted that in this embodiment, when the attachment 50 is added to the orthodontic aligner 10 at the first position, the first position refers to a position where there is no occlusal interference with the opposing tooth 60 of the first tooth. Before adjusting the position of the attachment 50 on the orthodontic aligner 10 to the first position, the attachment 50 is at a second position on the orthodontic aligner 10, where the second position refers to the position where the attachment 50 is located when there is occlusal interference with the opposing tooth 60 of the first tooth.

In another embodiment, when the attachment 50 is added at the first position on the orthodontic aligner, there may still be occlusal interference with the opposing tooth 60 of the first tooth, requiring further adjustment of the first position. During each adjustment, the position where the attachment 50 was before adjustment becomes the second position, and the position after adjustment becomes the new first position. For each adjustment, the priority level corresponding to the new first position is always lower than the priority level corresponding to the second position, until finally the attachment 50 is added to the orthodontic aligner 10 at the final first position where there is no occlusal interference with the opposing tooth 60 of the first tooth. It should be noted that during each adjustment, the priority level corresponding to the new first position may also equal the priority level corresponding to the second position, meaning that when there are multiple addition positions within the same priority level, if adding the attachment 50 at one position would cause occlusal interference with the opposing tooth 60 of the first tooth, the adjustment prioritizes other positions of equal priority.

Furthermore, as shown in FIG. 6, in this embodiment, when there is occlusal interference between the attachment 50 and the opposing tooth 60 of the first tooth, the position of the attachment 50 on the orthodontic aligner 10 is adjusted, and a direction of adjustment is toward a gingival margin. In some embodiments, the adjustment direction for different attachments 50 is not fixed and needs to be determined specifically based on the shape of the attachment 50 and the function it is intended to achieve.

It can be understood that during the continuous downgrading of the attachment's 50 installation position, simulated comparisons can be made between the second region where the attachment 50 is located and the first region. These two processes do not have a fixed order, but before eliminating occlusal interference, the edge cutting line 40 generated based on the comparison results is not the final edge cutting line 40. That is, before generating the final edge cutting line 40, it must be ensured that there is no occlusal interference between the attachment 50 and the opposing tooth 60 of the first tooth.

Furthermore, in this embodiment, the edge cutting line 40 generated based on the second region is specifically determined at a preset distance away from the position information of the second region. This position information of the second region specifically refers to an edge contour line of the second region, and the edge cutting line 40 is generated at a position 0.2mm to 2mm away from the edge contour line of the second region. That is, at least at the installation position of the attachment 50, the edge cutting line 40 is between 0.2mm and 2mm away from the edge contour of the second region. It should be noted that the position information of the second region is not limited to its edge contour line; in other embodiments, the position information can also be a central position of the second region or any information that can serve as a reference within the second region. Furthermore, in other embodiments, even when using the edge contour of the second region as reference information, the dimension between the edge cutting line 40 and the edge contour of the second region can be adjusted as needed, with no fixed restrictions.

Moreover, the finally generated edge cutting line 40 extends along a first direction of the first tooth and covers at least one tooth adjacent to the first tooth or an interdental space. The first direction can be a mesial direction or distal direction of the first tooth, or a direction determined based on other key points of the first tooth. For example, as shown in FIG. 7, when there is one first tooth, the finally generated edge cutting line 40 is between 0.2mm and 2mm away from the edge contour of the second region at the position of the first tooth, extends along the mesial and distal directions of the first tooth at the first tooth position, and covers one tooth adjacent to the first tooth. When the orthodontic aligner 10 needs to be fitted on other teeth, the edge cutting lines for the corresponding orthodontic aligner 10 on other teeth can be directly generated according to the corresponding gingival lines 30.

Furthermore, during the entire process of generating the edge cutting line 40, the portion of the dental model used for installing the attachment 50 or the entire dental model can be displayed, and the attachment 50 to be installed or the orthodontic aligner 10 fitted on the dental model, or both, can be displayed on the dental model through specific methods.

Optionally, after the edge cutting line 40 is generated according to the above method, the edge cutting line 40 can also be displayed, allowing operators to observe it more intuitively and thus making adjustments more convenient. Regarding how to display the edge cutting line 40, conventional technologies in the field can be used, for example, displaying the edge cutting of the orthodontic aligner 10 in real-time on a computer screen through specialized computer software; or displaying the edge cutting line 40 on a prepared dental model through special lighting.

Additionally, the method for generating the edge cutting line 40 also includes: obtaining a first instruction from a user, where the first instruction is used to indicate desired position information of the attachment 50 or desired position information of the orthodontic aligner 10 edge, and then using the first instruction and the second region as described above to generate a first cutting line. In practical operation, based on specific situations regarding patients or treatment, users can manually add the desired position of the attachment 50 or manually add the desired position of the orthodontic aligner 10 edge line to achieve better treatment effects.

For example, users can perform operations to add or move the attachment 50 on a display interface showing the dental model to determine the desired position of the attachment 50. After the user specifies the desired position of the attachment 50, the first instruction can be generated to indicate the desired position information of the attachment 50. The position information can be coordinate information, movement information, target position information of the attachment 50, or position information relative to the gingiva.

Another example is that users can perform operations to move the edge cutting line 40 on a display interface showing the dental model to determine the desired position of the edge cutting line 40. After the user specifies the desired position of the edge cutting line 40, the first instruction can be generated to indicate the desired position information of the edge cutting line 40. The position information can be coordinate information, movement information, target position information of the edge cutting line 40, or position information relative to the gingival line 30.

After obtaining the first instruction, the first cutting line is generated based on the desired position of the attachment 50 or the edge cutting line 40, and the second region.

Considering another possible scenario, after displaying the first cutting line, users can also adjust the first cutting line. For example, users can operate on the interface, which can involve adjusting either the position of the attachment 50 or the position of the first cutting line, where the interface can display information such as the generated first cutting line and attachment 50.

Meanwhile, the method for generating the edge cutting line 40 also includes: in response to a second instruction from the user, adjusting the generated first cutting line to a second cutting line, where the second instruction is used to indicate adjustment information for the first cutting line, and the second cutting line is used to generate a target orthodontic aligner 10.

For example, users can still adjust the position of the attachment 50 on the display interface showing the dental model. After the user specifies an updated position of the attachment 50, the second instruction can be generated to indicate the updated position information of the attachment 50. The position information can be coordinate information, movement information, target position information of the attachment 50, or position information relative to the gingiva.

Another example is that users can perform operations to move the first cutting line on the display interface showing the dental model to determine the target position of the first cutting line. After the user specifies the target position of the first cutting line, a second instruction can be generated to indicate the updated position information of the first cutting line. The position information can be coordinate information, movement information, target position information of the first cutting line, or position information relative to the gingival line 30.

After obtaining the second instruction, the first cutting line is adjusted to the second cutting line based on the target position of the attachment 50 or the first cutting line, and the second cutting line is used to generate the target orthodontic aligner 10.

In another possible implementation, the method for generating the edge cutting line 40 can include obtaining a dental model corresponding to the first tooth and, in response to the first instruction from the user, determining the second region corresponding to the attachment 50 and directly generating the edge cutting line 40 based on the second region. The dental model can be generated based on a three-dimensional model obtained by scanning the user's oral cavity, or based on two-dimensional images formed by photographing the user's oral cavity, or based on various types of image data obtained after scanning dental impression data.

It should be noted that the first instruction mentioned in this implementation can be the same as the first instruction in the above embodiment, meaning users can perform operations to add or move the attachment 50 on the display interface showing the dental model to determine the desired position of the attachment 50. After the user specifies the desired position of the attachment 50, the first instruction can be generated to indicate the desired position information of the attachment 50. The position information can be coordinate information, movement information, target position information of the attachment 50, or position information relative to the gingiva. After determining the position information of the attachment 50 based on the first instruction, the second region corresponding to the attachment 50 can be determined, and thus the edge cutting line 40 can be directly generated using the second region. The specific method for generating the edge cutting line based on the second region can refer to the above embodiment. However, the first instruction in this implementation can also be different from the first instruction in the above embodiment, as long as it can determine the position where the attachment 50 is to be added on the orthodontic aligner 10.

In this implementation, the portion of the dental model used for installing the attachment 50 can also be displayed, and the attachment 50 to be installed or the orthodontic aligner 10 fitted on the dental model, or both, can be displayed on the dental model through specific methods. Optionally, after the edge cutting line 40 is generated according to the above method, the edge cutting line 40 can be displayed, allowing operators to observe it more intuitively and thus making adjustments more convenient. Regarding how to display the edge cutting line 40, conventional technologies in the field can be used, for example, displaying the edge cutting of the orthodontic aligner 10 in real-time on a computer screen through specialized computer software; or displaying the edge cutting line 40 on the prepared dental model through special lighting.

An embodiment also provides an apparatus for generating an aligner cutting line, including:
A processing module, configured to identify a tooth contour of a dental model corresponding to at least one first tooth, and obtain a first region corresponding to the tooth contour;
A determining module, configured to determine a second region corresponding to an attachment, wherein the attachment is located at least partially on the orthodontic aligner corresponding to at least a portion of the at least one first tooth or an interdental space, wherein the orthodontic aligner is related to the dental model;
A generating module, configured to, when the first region cannot completely cover the second region, generate an edge cutting line for the orthodontic aligner through the processing module based on the second region, wherein the edge cutting line is configured to cut the orthodontic aligner.

In one possible implementation, the edge cutting line satisfies at least one of the following:
at least a portion of the edge cutting line is located below a first gingival line;
at least a portion of the edge cutting line intersects with the first gingival line;
at least a portion of the second region is located below the first gingival line;
at least a portion of the second region intersects with the first gingival line;
wherein the first gingival line includes a gingival line of the at least one first tooth, and/or at least a portion of a gingival line of at least one tooth adjacent to the at least one first tooth.

In one possible implementation, further including:
A processing module, configured to adjust the position of the attachment on the orthodontic aligner to a first position when there is occlusal interference between the attachment and an opposing tooth of the first tooth.

In one possible implementation, before adjusting the position of the attachment on the orthodontic aligner to the first position, the attachment is at a second position on the orthodontic aligner, wherein the second position is a position where the attachment is located when there is occlusal interference with the opposing tooth of the first tooth.

In one possible implementation, a priority of the first position is lower than or equal to a priority of the second position.

In one possible implementation, the priority of the position where the attachment is located is related to function information of the attachment.

In one possible implementation, further including:
A processing module, configured to adjust the position of the attachment on the orthodontic aligner when there is occlusal interference between the attachment and the opposing tooth of the first tooth, and a direction of adjustment is toward a gingival margin.

In one possible implementation, before generating the edge cutting line, there is no occlusal interference between the attachment and the opposing tooth of the first tooth.

In one possible implementation, the edge cutting line is determined at a preset distance away from position information of the second region.

In one possible implementation, the preset distance ranges from 0.2mm to 2mm.

In one possible implementation, the edge cutting line extends along a first direction of the first tooth and covers at least one tooth adjacent to the at least one first tooth or an interdental space.

In one possible implementation, further including:
A display module, configured to display at least a portion of the dental model; display at least one of the edge cutting line, the attachment, and the orthodontic aligner.

In one possible implementation, further including:
An acquisition module, configured to obtain a first instruction from a user, wherein the first instruction is configured to indicate desired position information of the attachment or desired position information of an aligner edge;

A processing module, configured to generate a first cutting line based on the first instruction and the second region.

In one possible implementation, further including:
A processing module, configured to, in response to a second instruction from the user, adjust the generated first cutting line to a second cutting line, wherein the second instruction is configured to indicate adjustment information of the first cutting line, and the second cutting line is configured to generate a target orthodontic aligner.

In one possible implementation, the second instruction is configured to indicate at least one of:
updating position information of the attachment;
an adjustment target position of the first cutting line.

The present application also provides an apparatus for generating an edge cutting line for an orthodontic aligner, including:
An acquisition module, configured to obtain a dental model corresponding to at least one first tooth;
A processing module, configured to, in response to a first instruction from a user and the dental model, determine a second region corresponding to the attachment, wherein the attachment is located at least partially on the orthodontic aligner corresponding to at least a portion of the at least one first tooth or an interdental space; wherein the first instruction is configured to indicate position information of the attachment; generate an edge cutting line based on the second region.

In one possible implementation, the edge cutting line satisfies at least one of the following:
at least a portion of the edge cutting line is located below a first gingival line;
at least a portion of the edge cutting line intersects with the first gingival line;
at least a portion of the second region is located below the first gingival line;
at least a portion of the second region intersects with the first gingival line;
wherein the first gingival line includes a gingival line of the at least one first tooth, and/or at least a portion of a gingival line of at least one tooth adjacent to the at least one first tooth.

In one possible implementation, further including:
A processing module, configured to adjust the position of the attachment on the orthodontic aligner to a first position when there is occlusal interference between the attachment and an opposing tooth of the first tooth.

In one possible implementation, before adjusting the position of the attachment on the orthodontic aligner to the first position, the attachment is at a second position on the orthodontic aligner, wherein the second position is a position where the attachment is located when there is occlusal interference with the opposing tooth of the first tooth.

In one possible implementation, a priority of the first position is lower than or equal to a priority of the second position.

In one possible implementation, the priority of the position where the attachment is located is related to function information of the attachment.

In one possible implementation, further including:
A processing module, configured to adjust the position of the attachment on the orthodontic aligner when there is occlusal interference between the attachment and the opposing tooth of the first tooth, and a direction of adjustment is toward a gingival margin.

In one possible implementation, before generating the edge cutting line, there is no occlusal interference between the attachment and the opposing tooth of the first tooth.

In one possible implementation, the edge cutting line is determined at a preset distance away from position information of the second region.

In one possible implementation, the preset distance ranges from 0.2mm to 2mm.

In one possible implementation, the edge cutting line extends along a first direction of the first tooth and covers at least one tooth adjacent to the at least one first tooth or an interdental space.

In one possible implementation, further including:
A display module, configured to display at least a portion of the dental model; display at least one of the edge cutting line, the attachment, and the orthodontic aligner.

In one possible implementation,
A processing module, configured to, in response to a second instruction from the user, adjust the generated first cutting line to a second cutting line, wherein the second instruction is configured to indicate adjustment information of the first cutting line, and the second cutting line is configured to generate a target orthodontic aligner.

In one possible implementation, the second instruction is configured to indicate at least one of:updating position information of the attachment;an adjustment target position of the first cutting line.

An embodiment also provides an electronic device including a memory, a processor, and a computer program stored in the memory and executable on the processor, wherein when the processor executes the program, it implements any of the possible methods in the above embodiments.

As shown in FIG. 8, at the hardware level, the electronic device includes a processor, an internal bus, a network interface, memory, and non-volatile storage, and may of course include other hardware required for business needs. The processor reads corresponding computer programs from the non-volatile storage into memory and runs them to implement the above-mentioned oral model display method or multi-planar reconstruction method. Of course, in addition to software implementation, this specification does not exclude other implementation methods, such as logic devices or combinations of software and hardware, which means that the execution subject of the following processing flow is not limited to various logical units, but can also be hardware or logic devices.

For convenience of description, the above apparatus is described by dividing functions into various units for separate description. Of course, when implementing this specification, the functions of each unit can be implemented in one or more software and/or hardware.

Technical personnel in the field should understand that the embodiments of the present application can be provided as methods, systems, or computer program products. Therefore, the present application can take the form of complete hardware embodiments, complete software embodiments, or embodiments combining software and hardware aspects. Moreover, the present application can take the form of a computer program product implemented on one or more computer-usable storage media (including but not limited to disk storage, CD-ROM, optical storage, etc.) containing computer-usable program code.

In a typical configuration, a computing device includes one or more processors (CPUs), input/output interfaces, network interfaces, and memory.

The memory can include volatile memory, such as Random Access Memory (RAM) and/or cache memory, and can further include Read-Only Memory (ROM).

The memory can also include program tools (or utilities) with a set (at least one) of program modules, such program modules including but not limited to: operating systems, one or more applications, other program modules, and program data, each of these examples or certain combinations may include implementations of network environments.

The processor executes various functional applications and data processing by running computer programs stored in memory, such as the methods in the above embodiments.

Input and/or output devices can include scanning devices, camera interfaces, input devices (e.g., mouse, keyboard, etc.), display devices (e.g., monitors), printers, and/or one or more other input devices. The input/output interface can receive executable instructions and/or data that can be stored in data storage devices (e.g., memory), for example, for receiving or storing oral model data or target files.

In some embodiments, the scanning device can be configured to scan one or more physical dental models of a patient's dentition. In one or more embodiments, the scanning device can be configured to directly scan a patient's dentition and/or dental appliances. The scanning device can be configured to input data into the computing device.

In some embodiments, the camera interface can receive input from imaging devices (e.g., 2D or 3D imaging devices) such as digital cameras, printed photo scanners, and/or other suitable imaging devices. For example, input from imaging devices can be stored in memory.

The processor can execute instructions to provide displays of oral models on the display, etc. For example, the computing device can be configured to allow treatment professionals or other users to input treatment goals. Received inputs can be sent as data to the processor and/or can be stored in memory.

This connectivity can allow input and/or output of data and/or instructions and other types of information. Some embodiments can be distributed among various computing devices within one or more networks and used for collecting, computing, and/or analyzing any of the methods mentioned in this application.

It should be noted that although several units/modules or sub-units/modules of the electronic device are mentioned in the detailed description above, such division is only exemplary and not mandatory. In fact, according to the implementation of this application, the features and functions of two or more units/modules described above can be embodied in one unit/module. Conversely, the features and functions of one unit/module described above can be further divided to be embodied by multiple units/modules.

An embodiment also relates to an orthodontic aligner 10, which is configured to be fitted on teeth, including an aligner body and an attachment 50, where the attachment 50 is specifically mounted on the aligner body. When the aligner body is fitted on teeth, the attachment 50 acts on specific teeth in a specific manner to achieve orthodontic correction of specific teeth. The edge cutting line of this aligner body is obtained using the method for generating an edge cutting line 40 for an orthodontic aligner as described above.

As described above for the method for generating an edge cutting line 40 for an orthodontic aligner, this embodiment also relates to a computer-readable medium, specifically a non-transitory computer-readable medium, which stores instructions executable by a processor to cause a computing device to perform the generation method described above.

Technical personnel in the field should understand that the embodiments of this specification can be provided as methods, systems, or computer program products. Therefore, this specification can take the form of complete hardware embodiments, complete software embodiments, or embodiments combining software and hardware aspects. Moreover, this specification can take the form of a computer program product implemented on one or more computer-usable storage media (including but not limited to disk storage, CD-ROM, optical storage, etc.) containing computer-usable program code.

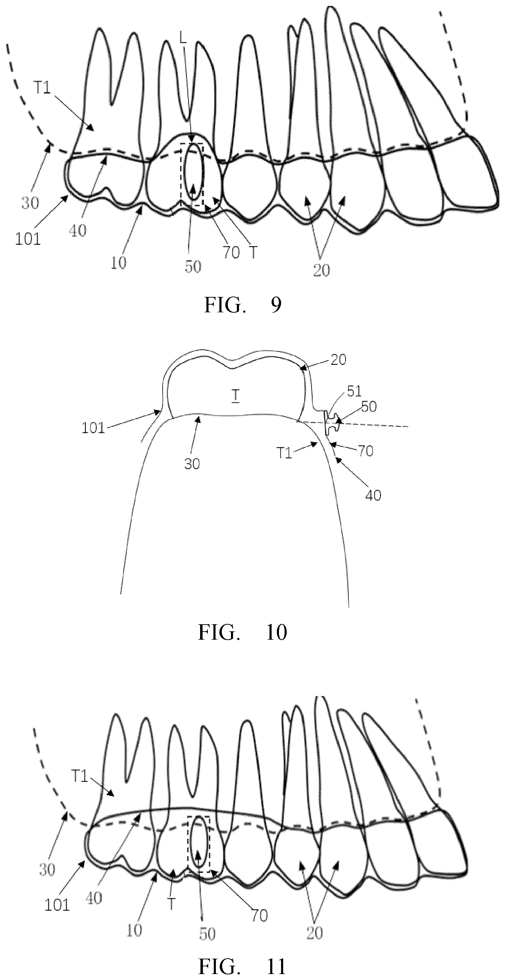

An embodiment of the present application provides an orthodontic aligner 10. Combined with FIGs. 9 and 10, the orthodontic aligner 10 includes an aligner body 101 and an attachment 50 mounted on the aligner body 101, wherein the aligner body 101 includes a second region 70 for adding the attachment 50. When the orthodontic aligner 10 is fitted on teeth T, a portion of the second region 70 is located below a first gingival line 30 of the teeth T, and another portion is located above the first gingival line 30 of the teeth T.

The teeth T uses the first gingival line 30 as a boundary line, where above the first gingival line 30 is a direction of crowns 20 of the teeth T, and below the first gingival line 30 is a direction of gingiva T1 of the teeth T.

In this embodiment, the second region 70 extends from the crowns 20 region above the first gingival line 30 to the gingival T1 region below the first gingival line 30. The second region 70 is relatively large, providing more space for attachment 50 installation.

Optionally, when the orthodontic aligner 10 is fitted on the teeth T, part of a bottom surface 51 of the attachment 50 connecting with the second region 70 corresponds to a region below the first gingival line 30, and part corresponds to a region above the first gingival line 30. That is, an installation position of the attachment 50 extends from the crowns 20 region above the first gingival line 30 to the gingival T1 region below the first gingival line 30, providing a larger installation position for the attachment 50 and greater fixation strength between the attachment 50 and the second region 70.

Optionally, the bottom surface 51 of the attachment 50 connects with the second region 70, and the bottom surface 51 covers part or all of the second region 70.

When the bottom surface 51 covers part of the second region 70, it can be located in a middle area, lower area, or upper area of the second region 70, and the projection of the attachment 50 within the second region 70 can be within or extend beyond an outer contour of the second region 70.

When the bottom surface 51 covers the entire second region 70, an outer contour of the bottom surface 51 coincides with the outer contour of the second region 70, and the projection of the attachment 50 within the second region 70 can be within or extend beyond the outer contour of the second region 70.

Optionally, interdental spaces are formed between the crowns 20 of adjacent teeth. When the orthodontic aligner 10 is fitted on teeth, the second region 70 corresponds to the crown 20 of at least one first tooth T or interdental space.

When there is one first tooth T, the second region 70 corresponds to the crown 20 and gingiva T1 of the first tooth. When there are multiple first teeth T, the second region 70 corresponds to the crowns 20, gingiva T1, and interdental spaces of multiple first teeth T.

Optionally, when the orthodontic aligner 10 is fitted on the teeth T, the attachment 50 is located at least partially on the orthodontic aligner 10 corresponding to at least a portion of at least one first tooth T or interdental space.

When there is one first tooth T, the attachment 50 corresponds to the crown 20 and gingiva T1 of the first tooth T. When there are multiple first teeth T, the attachment 50 corresponds to the crowns 20, gingiva T1, and interdental spaces of multiple first teeth T.

Optionally, as shown in FIG. 9, the orthodontic aligner 10 includes an edge cutting line 40, which is located below the first gingival line 30 only at the portion of the first tooth T, while the edge cutting line 40 substantially coincides with the first gingival line 30 at positions of other teeth.

FIG. 9 shows an example with one first tooth T, where the second region 70 corresponds to the crown 20 and gingiva T1 of the first tooth T. The edge cutting line 40 extends below the first gingival line 30 into the gingival T1 region only at the first tooth T, while substantially coinciding with the first gingival line 30 at other teeth.

In FIG. 9, the edge cutting line 40 can also extend below the first gingival line 30 into the gingival T1 region only at the second region 70, while substantially coinciding with the first gingival line 30 in areas of the first tooth T outside the second region 70 and at other teeth.

Optionally, as shown in FIG. 11, the orthodontic aligner 10 includes an edge cutting line 40, which is located below the first gingival line 30 at portions of both the first tooth T and adjacent teeth.

FIG. 11 shows an example with one first tooth T, where the second region 70 corresponds to the crown 20 and gingiva T1 of the first tooth T. The edge cutting line 40 not only extends below the first gingival line 30 into the gingival T1 region at the first tooth T but also extends below the first gingival line 30 into the gingival T1 region at the adjacent teeth on both left and right sides. In this case, the edge cutting line 40 extending below the first gingival line 30 spans three teeth.

Optionally, a preset distance L between the edge cutting line 40 and a corresponding edge contour line of the second region 70 ranges from 0.2mm to 2mm.

The preset distance L is a vertical distance between the edge cutting line 40 and the edge contour line of the second region 70, controlled through the preset distance L.

Optionally, when the orthodontic aligner 10 is fitted on the teeth and in occlusion with opposing teeth, the attachment 50 is spaced apart from the opposing teeth, meaning there is no interference between the attachment 50 and opposing teeth when in occlusion.

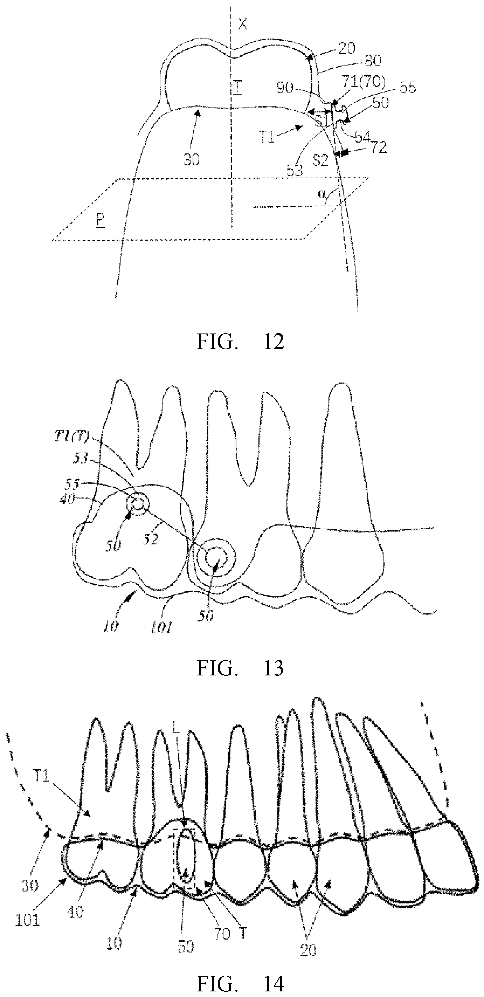

Optionally, as shown in FIG. 12, the tooth includes a crown 20 located above the first gingival line 30 and gingiva T1 located below the first gingival line 30. The second region 70 includes a first end portion 71 corresponding to the crown 20 and a second end portion 72 corresponding to the gingiva T1. When the orthodontic aligner 10 is fitted on the teeth, there is a first distance S1 between the first end portion 71 and the crown 20, and a second distance S2 between the second end portion 72 and the gingiva T1, where the first distance S1 is greater than the second distance S2.

"The first distance S1 is greater than the second distance S2" means that an area of the second region 70 near an incisal edge (or occlusal surface) of the tooth is farther from the tooth compared to an area away from the incisal edge (or occlusal surface), equivalent to locally lifting and raising the originally tooth-fitting second region 70 outward, making the second region 70 extend roughly along direction X perpendicular to an occlusal plane P, i.e., the second region 70 tends toward a vertical state.

The attachment 50 can be a traction buckle, traction hook, tubular attachment, or groove-shaped attachment. Compared to installing the attachment on a horizontally oriented second region, an attachment 50 installed on the vertically oriented second region 70 is beneficial for fitting traction members (e.g., elastic bands), preventing the traction members from easily detaching from the attachment 50, and ensuring stable force application by the traction members.

Optionally, the second region 70 is a plane, and an angle α between the plane of the second region 70 and the occlusal plane P ranges from 60° to 90°, meaning the plane of the second region 70 extends roughly along direction X perpendicular to the occlusal plane P, but not necessarily absolutely perpendicular.

Optionally, the second end portion 72 is tightly fitted to the gingiva T1. The second region 70 is only lifted and raised outward at the first end portion 71 and areas adjacent to it, the second end portion 72 is not pulled outward or raised, and the second end portion 72 remains tightly fitted to the gingiva T1, which can improve the overall wrapping performance of the orthodontic aligner 10 on the teeth.

Optionally, the aligner body 101 further includes a third region 80 and a connecting region 90 connecting the third region 80 and the second region 70. The third region 80 is tightly fitted to the crown 20, and the connecting region 90 forms a bend between the second region 70 and the third region 80.

"The connecting region 90 forms a bend between the second region 70 and the third region 80" means that the connecting region 90 forms a bend with the second region 70 and also forms a bend with the third region 80, achieving the outward lifting and raising of the second region 70.

The connecting region 90 extends roughly horizontally, the second region 70 extends roughly vertically, and the third region 80 is roughly perpendicular to the connecting region 90, while the connecting region 90 is roughly perpendicular to the second region 70, but this is not limiting. The third region 80, connecting region 90, and second region 70 can also have other bending forms.

As shown in FIG. 13, it is a schematic diagram of two attachments 50 on the same arch connected by a traction member 52. Taking the attachment 50 as a traction buckle for example, the attachment 50 includes a bottom portion 53 connecting to the second region 70, a connecting rod 54 connecting to the bottom portion 53, and a top portion 55 located on a side of the connecting rod 54 away from the bottom portion 53, with the traction member 52 fitted on the connecting rod 54.

When the second region 70 extends roughly along direction X perpendicular to the occlusal plane P, the extension direction of the connecting rod 54 is roughly perpendicular to the buccal surface of the teeth, while the extension direction of the traction member 52 is roughly parallel to the buccal surface of the tooth, which can prevent the traction member 52 from detaching from the connecting rod 54 and ensure stable force application by the traction member 52.

When the two attachments 50 are on different arches, the second region 70 extending roughly along direction X perpendicular to the occlusal plane P can also help prevent the traction member 52 from detaching from the connecting rod 54 and ensure stable force application by the traction member 52.

It should be noted that a junction between the crowns 20 and gingiva T1 is a relatively steep slope, where the second region 70 is more likely to tend toward a horizontal state. Therefore, lifting and raising this part of the second region 70 outward can effectively prevent the normal use of attachment 50 from being affected by angle issues of the second region 70.

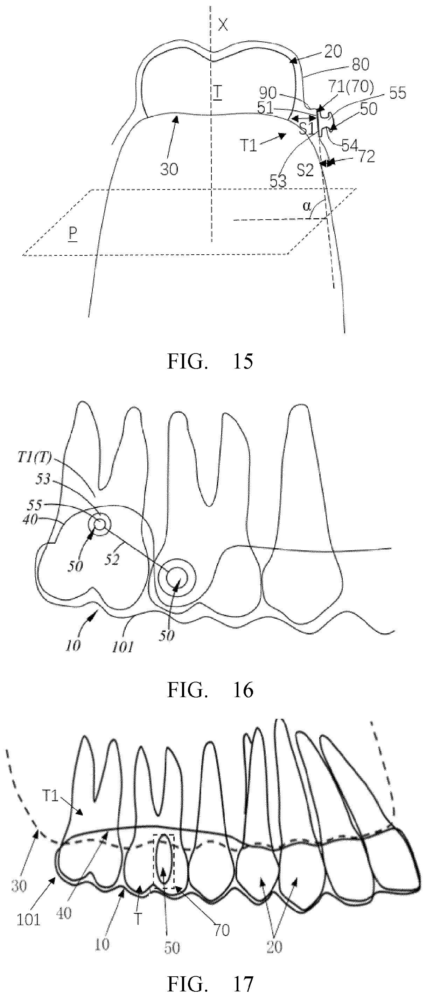

Another embodiment of the present application provides an orthodontic aligner 10. As shown in FIGs. 14 and 15, the orthodontic aligner 10 includes an aligner body 101 and an attachment 50 mounted on the aligner body 101, wherein the aligner body 101 includes a second region 70 for adding the attachment 50. When the orthodontic aligner 10 is fitted on teeth T, the second region 70 is close to a first gingival line 30 of the teeth, or the second region 70 intersects with the first gingival line 30.

The teeth T uses the first gingival line 30 as a boundary line, where above the first gingival line 30 is a direction of crowns 20 of the teeth T, and below the first gingival line 30 is a direction of gingiva T1 of the teeth T.

"The second region 70 is close to the first gingival line 30 of the tooth" means that the second region 70 is in an area adjacent to the first gingival line 30, while "the second region 70 intersects with the first gingival line 30" means that the first gingival line 30 passes through the second region 70. In any case, the second region 70 is in an area away from an incisal edge (or occlusal surface) of the teeth.

Optionally, a portion of the second region 70 is located below the first gingival line 30 of the teeth T and another portion is located above the first gingival line 30; or, the second region 70 is entirely located below the first gingival line 30, or the second region 70 is entirely located above the first gingival line 30. That is, the second region 70 can be located on one side of the first gingival line 30, or on both sides. This embodiment takes the example where a portion of the second region 70 is located below the first gingival line 30 of the teeth T and another portion is located above. The second region 70 extends from the crowns 20 region above the first gingival line 30 to the gingival T1 region below the first gingival line 30, providing a larger second region 70 that offers more space for attachment 50 installation.

In a direction perpendicular to an occlusal plane P, the second region 70 includes oppositely disposed first end portion 71 and second end portion 72, where there is a first distance S1 between the first end portion 71 and the tooth T, and a second distance S2 between the second end portion 72 and the tooth T, with the first distance S1 being greater than the second distance S2.

"The first distance S1 is greater than the second distance S2" means that an area of the second region 70 near the incisal edge (or occlusal surface) of the tooth is farther from the tooth compared to an area away from the incisal edge (or occlusal surface), equivalent to locally lifting and raising the originally tooth-fitting second region 70 outward, making the second region 70 extend roughly along direction X perpendicular to the occlusal plane P, i.e., the second region 70 tends toward a vertical state.

The attachment 50 can be a traction buckle, traction hook, tubular attachment, or groove-shaped attachment. Compared to installing the attachment on a horizontally oriented second region, an attachment 50 installed on the vertically oriented second region 70 is beneficial for fitting traction members (e.g., elastic bands), preventing the traction members from easily detaching from the attachment 50, and ensuring stable force application by the traction members.

When a portion of the second region 70 is located below the first gingival line 30 of the tooth T and another portion is located above, the first end portion 71 corresponds to the crown 20, with the first distance S1 between the first end portion 71 and the crown 20, while the second end portion 72 corresponds to the gingiva T1, with the second distance S2 between the second end portion 72 and the gingiva T1.

Optionally, the second region 70 is a plane, and an angle α between the plane of the second region 70 and the occlusal plane P ranges from 60° to 90°, meaning the plane of the second region 70 extends roughly along direction X perpendicular to the occlusal plane P, but not necessarily absolutely perpendicular.

Optionally, the second end portion 72 is tightly fitted to the gingiva T1. The second region 70 is only lifted and raised outward at the first end portion 71 and areas adjacent to it, the second end portion 72 is not pulled outward or raised, and the second end portion 72 remains tightly fitted to the gingiva T1, which can improve the overall wrapping performance of the orthodontic aligner 10 on the teeth.

Optionally, the aligner body 101 further includes a third region 80 and a connecting region 90 connecting the third region 80 and the second region 70. The third region 80 is tightly fitted to the crowns 20, and the connecting region 90 forms a bend between the second region 70 and the third region 80.

"The connecting region 90 forms a bend between the second region 70 and the third region 80" means that the connecting region 90 forms a bend with the second region 70 and also forms a bend with the third region 80, achieving the outward lifting and raising of the second region 70.

The connecting region 90 extends roughly horizontally, the second region 70 extends roughly vertically, and the third region 80 is roughly perpendicular to the connecting region 90, while the connecting region 90 is roughly perpendicular to the second region 70, but this is not limiting. The third region 80, connecting region 90, and second region 70 can also have other bending forms.

As shown in FIG. 16, it is a schematic diagram of two attachments 50 on the same arch connected by a traction member 52. Taking the attachment 50 as a traction buckle for example, the attachment 50 includes a bottom portion 53 connecting to the second region 70, a connecting rod 54 connecting to the bottom portion 53, and a top portion 55 located on a side of the connecting rod 54 away from the bottom portion 53, with the traction member 52 fitted on the connecting rod 54.

When the second region 70 extends roughly along direction X perpendicular to the occlusal plane P, the extension direction of the connecting rod 54 is roughly perpendicular to the buccal surface of the tooth, while the extension direction of the traction member 52 is roughly parallel to the buccal surface of the tooth, which can prevent the traction member 52 from detaching from the connecting rod 54 and ensure stable force application by the traction member 52.

When the two attachments 50 are on different arches, the second region 70 extending roughly along direction X perpendicular to the occlusal plane P can also help prevent the traction member 52 from detaching from the connecting rod 54 and ensure stable force application by the traction member 52.

It should be noted that the junction between the crown 20 and gingiva T1 is a relatively steep slope, where the second region 70 is more likely to tend toward a horizontal state. Therefore, lifting and raising this part of the second region 70 outward can effectively prevent the normal use of attachment 50 from being affected by angle issues of the second region 70.

Optionally, the bottom surface 51 of the attachment 50 connects with the second region 70, and the bottom surface 51 covers part or all of the second region 70.

When the bottom surface 51 covers part of the second region 70, it can be located in a middle area, lower area, or upper area of the second region 70, and the projection of the attachment 50 within the second region 70 can be within or extend beyond an outer contour of the second region 70.

When the bottom surface 51 covers the entire second region 70, an outer contour of the bottom surface 51 coincides with the outer contour of the second region 70, and the projection of the attachment 50 within the second region 70 can be within or extend beyond the outer contour of the second region 70.

Optionally, as shown in FIG. 14, the second region 70 partially extends below the first gingival line 30, and the orthodontic aligner 10 includes an edge cutting line 40, which is located below the first gingival line 30 only at the portion of the second region 70, while other parts of the edge cutting line 40 substantially coincide with the first gingival line 30.

FIG. 14 shows an example where the second region 70 corresponds to the crown 20 and gingiva T1 of one tooth T, where the edge cutting line 40 extends below the first gingival line 30 into the gingival T1 region only at tooth T, while substantially coinciding with the first gingival line 30 at other teeth.

In FIG. 14, the edge cutting line 40 can also extend below the first gingival line 30 into the gingival T1 region only at the second region 70, while substantially coinciding with the first gingival line 30 in areas of the tooth T outside the second region 70 and at other teeth.

Optionally, as shown in FIG. 17, the orthodontic aligner 10 includes the edge cutting line 40, which is located below the first gingival line 30 at portions of both the second region 70 and teeth adjacent to the second region 70.

FIG. 17 shows an example where the second region 70 corresponds to the crown 20 and gingiva T1 of one tooth T, where the edge cutting line 40 not only extends below the first gingival line 30 into the gingival T1 region at the tooth T with the second region 70, but also extends below the first gingival line 30 into the gingival T1 region at the adjacent teeth on both left and right sides. In this case, the edge cutting line 40 extending below the first gingival line 30 spans three teeth.

Optionally, the preset distance L between the edge cutting line 40 and the corresponding edge contour line of the second region 70 ranges from 0.2mm to 2mm.

The preset distance L is the vertical distance between the edge cutting line 40 and the edge contour line of the second region 70, controlled through the preset distance L.

Optionally, when the orthodontic aligner 10 is fitted on the teeth and in occlusion with opposing teeth, the attachment 50 is spaced apart from the opposing teeth, meaning there is no interference between the attachment 50 and opposing teeth when in occlusion.

Although specific embodiments of the present application have been described above, persons skilled in the art should understand that these are only examples, and the scope of protection of the present application is defined by the appended claims. Persons skilled in the art may make various changes or modifications to these embodiments without departing from the principle and essence of the present application, but all these changes and modifications fall within the scope of protection of the present application.

## Claims

1. A method for generating an edge cutting line for an orthodontic aligner, **characterized in that** the generating method comprises:
identifying a tooth contour of a dental model corresponding to at least one first tooth, and obtaining a first region corresponding to the tooth contour;
determining a second region corresponding to an attachment, wherein the attachment is located at least partially on the orthodontic aligner corresponding to at least a portion of the at least one first tooth or an interdental space, wherein the orthodontic aligner is related to the dental model;
when the first region cannot completely cover the second region, generating an edge cutting line for the orthodontic aligner based on the second region, wherein the edge cutting line is configured to cut the orthodontic aligner.

2. The method for generating an edge cutting line for an orthodontic aligner according to claim 1, **characterized in that** the edge cutting line satisfies at least one of the following:
at least a portion of the edge cutting line is located below a first gingival line;
at least a portion of the edge cutting line intersects with the first gingival line;
at least a portion of the second region is located below the first gingival line;
at least a portion of the second region intersects with the first gingival line;
wherein the first gingival line comprises a gingival line of the at least one first tooth, and/or at least a portion of a gingival line of at least one tooth adjacent to the at least one first tooth.

3. The method for generating an edge cutting line for an orthodontic aligner according to any one of claims 1-2, **characterized in that** the generating method further comprises:
when there is occlusal interference between the attachment and an opposing tooth of the first tooth, adjusting a position of the attachment on the orthodontic aligner to a first position.

4. The method for generating an edge cutting line for an orthodontic aligner according to claim 3, **characterized in that** before adjusting the position of the attachment on the orthodontic aligner to the first position, the attachment is at a second position on the orthodontic aligner, wherein the second position is a position where the attachment is located when there is occlusal interference between the attachment and the opposing tooth of the first tooth.

5. The method for generating an edge cutting line for an orthodontic aligner according to claim 4, **characterized in that** a priority of the first position is lower than or equal to a priority of the second position.

6. The method for generating an edge cutting line for an orthodontic aligner according to claim 5, **characterized in that** the priority of the position where the attachment is located is related to function information of the attachment.

7. The method for generating an edge cutting line for an orthodontic aligner according to any one of claims 1-6, **characterized in that** the generating method further comprises:
when there is occlusal interference between the attachment and the opposing tooth of the first tooth, adjusting the position of the attachment on the orthodontic aligner, and the direction of adjustment is toward a gingival margin.

8. The method for generating an edge cutting line for an orthodontic aligner according to any one of claims 1-7, **characterized in that** before generating the edge cutting line, there is no occlusal interference between the attachment and the opposing tooth of the first tooth.

9. The method for generating an edge cutting line for an orthodontic aligner according to any one of claims 1-8, **characterized in that** the edge cutting line is determined at a preset distance away from position information of the second region.

10. The method for generating an edge cutting line for an orthodontic aligner according to claim 9, **characterized in that** the preset distance ranges from 0.2mm to 2mm.

11. The method for generating an edge cutting line for an orthodontic aligner according to any one of claims 1-10, **characterized in that** the edge cutting line extends along a first direction of the first tooth, and at least covers at least one tooth adjacent to the at least one first tooth or an interdental space.

12. The method for generating an edge cutting line for an orthodontic aligner according to any one of claims 1-11, **characterized in that** the generating method further comprises:
displaying at least a portion of the dental model;
displaying at least one of the edge cutting line, the attachment, and the orthodontic aligner.

13. The method for generating an edge cutting line for an orthodontic aligner according to any one of claims 1-12, **characterized in that** the generating method further comprises:
obtaining a first instruction from a user, wherein the first instruction is configured to indicate desired position information of the attachment or desired position information of an orthodontic aligner edge;
wherein generating the edge cutting line based on the second region comprises: generating a first cutting line based on the first instruction and the second region.

14. The method for generating an edge cutting line for an orthodontic aligner according to claim 13, **characterized in that** the generating method further comprises:
in response to a second instruction from the user, adjusting the generated first cutting line to a second cutting line, wherein the second instruction is configured to indicate adjustment information of the first cutting line, and the second cutting line is configured to generate a target orthodontic aligner.

15. The method for generating an edge cutting line for an orthodontic aligner according to claim 14, **characterized in that** the second instruction is configured to indicate at least one of:
updating position information of the attachment;
an adjustment target position of the first cutting line.

16. A method for generating an edge cutting line for an orthodontic aligner, **characterized in that** the generating method comprises:
obtaining a dental model corresponding to at least one first tooth;
in response to a first instruction from a user and the dental model, determining a second region corresponding to an attachment, wherein the attachment is located at least partially on an orthodontic aligner corresponding to at least a portion of the at least one first tooth or an interdental space; wherein the first instruction is configured to indicate position information of the attachment;
generating an edge cutting line based on the second region.

17. The method for generating an edge cutting line for an orthodontic aligner according to claim 16, **characterized in that** the edge cutting line satisfies at least one of the following:
at least a portion of the edge cutting line is located below a first gingival line;
at least a portion of the edge cutting line intersects with the first gingival line;
at least a portion of the second region is located below the first gingival line;
at least a portion of the second region intersects with the first gingival line;
wherein the first gingival line comprises a gingival line of the at least one first tooth, and/or at least a portion of a gingival line of at least one tooth adjacent to the at least one first tooth.

18. The method for generating an edge cutting line for an orthodontic aligner according to any one of claims 16-17, **characterized in that** before generating the edge cutting line, there is no occlusal interference between the attachment and an opposing tooth of the first tooth.

19. The method for generating an edge cutting line for an orthodontic aligner according to any one of claims 16-18, **characterized in that** the edge cutting line is determined at a preset distance away from position information of the second region.

20. The method for generating an edge cutting line for an orthodontic aligner according to claim 19, **characterized in that** the preset distance ranges from 0.2mm to 2mm.

21. The method for generating an edge cutting line for an orthodontic aligner according to any one of claims 16-20, **characterized in that** the edge cutting line extends along a first direction of the first tooth, and at least covers at least one tooth adjacent to the at least one first tooth or an interdental space.

22. The method for generating an edge cutting line for an orthodontic aligner according to any one of claims 16-21, **characterized in that** the generating method further comprises:
displaying at least a portion of the dental model;
displaying at least one of the edge cutting line, the attachment, and the orthodontic aligner.

23. An orthodontic aligner configured to be fitted on teeth, **characterized in that** the orthodontic aligner comprises an aligner body and an attachment mounted on the aligner body;
wherein an edge of the orthodontic aligner is determined based on the method for generating an edge cutting line for an orthodontic aligner according to any one of claims 1-15;
or, the edge of the orthodontic aligner is determined based on the method for generating an edge cutting line for an orthodontic aligner according to any one of claims 16-22.

24. A computer-readable medium, **characterized in that** the computer-readable medium stores instructions executable by a processor to cause a computing device to perform a method, wherein the method comprises the method for generating an edge cutting line for an orthodontic aligner according to any one of claims 1-15;
or, the method comprises the method for generating an edge cutting line for an orthodontic aligner according to any one of claims 16-22.

25. An orthodontic aligner, **characterized in that** the orthodontic aligner comprises an aligner body and an attachment mounted on the aligner body, wherein the aligner body comprises a second region for adding the attachment, and when the orthodontic aligner is fitted on teeth, a portion of the second region is located below a first gingival line of teeth and another portion is located above the first gingival line of teeth.

26. The orthodontic aligner according to claim 25, **characterized in that** when the orthodontic aligner is fitted on teeth, a portion of a bottom surface of the attachment connecting with the second region corresponds to a region below the first gingival line, and another portion corresponds to a region above the first gingival line.

27. The orthodontic aligner according to claim 25, **characterized in that** when the orthodontic aligner is fitted on teeth, the second region corresponds to a crown of at least one first tooth or an interdental space, when there is one first tooth, the second region corresponds to the crown and gingiva of the first tooth, when there are multiple first teeth, the second region corresponds to crowns, gingiva, and interdental spaces of the multiple first teeth.

28. The orthodontic aligner according to claim 25, **characterized in that** when the orthodontic aligner is fitted on teeth, the attachment is located at least partially on the orthodontic aligner corresponding to at least a portion of the at least one first tooth or an interdental space, when there is one first tooth, the attachment corresponds to the crown and gingiva of the first tooth, when there are multiple first teeth, the attachment corresponds to crowns, gingiva, and interdental spaces of the multiple first teeth.

29. The orthodontic aligner according to claim 27 or 28, **characterized in that** the orthodontic aligner comprises an edge cutting line, wherein the edge cutting line is located below the first gingival line only at the portion of the first tooth, and the edge cutting line substantially coincides with the first gingival line at portions of other teeth.

30. The orthodontic aligner according to claim 27 or 28, **characterized in that** the orthodontic aligner comprises an edge cutting line, wherein the edge cutting line is located below the first gingival line at the portion of the first tooth and portions of other teeth adjacent to the first tooth.

31. The orthodontic aligner according to claim 25, **characterized in that** the teeth comprise crowns located above the first gingival line and gingiva located below the first gingival line, the second region comprises a first end portion corresponding to the crown and a second end portion corresponding to the gingiva, when the orthodontic aligner is fitted on teeth, there is a first distance between the first end portion and the crown, and there is a second distance between the second end portion and the gingiva, wherein the first distance is greater than the second distance.

32. The orthodontic aligner according to claim 31, **characterized in that** the second end portion is tightly fitted to the gingiva.

33. An orthodontic aligner, **characterized in that** the orthodontic aligner comprises an aligner body and an attachment mounted on the aligner body, wherein the aligner body comprises a second region for adding the attachment, when the orthodontic aligner is fitted on teeth, the second region is close to a first gingival line of teeth, or the second region intersects with the first gingival line, in a direction perpendicular to an occlusal plane, the second region comprises oppositely disposed first end portion and second end portion, there is a first distance between the first end portion and the teeth, and there is a second distance between the second end portion and the teeth, wherein the first distance is greater than the second distance.

34. The orthodontic aligner according to any one of claims 31-33, **characterized in that** the aligner body further comprises a third region and a connecting region connecting the third region and the second region, wherein the third region is tightly fitted to the crown, and the connecting region forms a bend between the second region and the third region.

35. The orthodontic aligner according to claim 25 or 33, **characterized in that** the second region is a plane.

36. The orthodontic aligner according to claim 35, **characterized in that** an angle between the plane of the second region and the occlusal plane ranges from 60° to 90°.

37. The orthodontic aligner according to claim 25 or 33, **characterized in that** a bottom surface of the attachment connects with the second region, and the bottom surface covers part or all of the second region.

38. The orthodontic aligner according to claim 25 or 33, **characterized in that** the attachment is a traction buckle, a traction hook, a tubular attachment, or a groove-shaped attachment.

39. The orthodontic aligner according to claim 25 or 33, **characterized in that** the second region partially extends below the first gingival line, the orthodontic aligner comprises an edge cutting line, and a preset distance between the edge cutting line and an outer contour line of the corresponding second region ranges from 0.2mm to 2mm.

40. The orthodontic aligner according to claim 25 or 33, **characterized in that** when the orthodontic aligner is fitted on teeth and in occlusion with opposing teeth, the attachment is spaced apart from the opposing teeth.

41. The orthodontic aligner according to claim 33, **characterized in that** a portion of the second region is located below the first gingival line of teeth and another portion is located above the first gingival line of teeth; or the second region is entirely located below the first gingival line; or the second region is entirely located above the first gingival line.

42. The orthodontic aligner according to claim 41, **characterized in that** the teeth comprise crowns located above the first gingival line and gingiva located below the first gingival line, wherein the first end portion corresponds to the crown and there is the first distance between the first end portion and the crown, and the second end portion corresponds to the gingiva and there is the second distance between the second end portion and the gingiva.

43. The orthodontic aligner according to claim 42, **characterized in that** the second end portion is tightly fitted to the gingiva.

44. The orthodontic aligner according to claim 33, **characterized in that** the second region partially extends below the first gingival line, the orthodontic aligner comprises an edge cutting line, wherein the edge cutting line is located below the first gingival line only at the portion of the second region, and other portions of the edge cutting line substantially coincide with the first gingival line.

45. The orthodontic aligner according to claim 33, **characterized in that** the second region partially extends below the first gingival line, the orthodontic aligner comprises an edge cutting line, wherein the edge cutting line is located below the first gingival line at the portion of the second region and portions of other teeth adjacent to the second region.
